Europäisches Patentamt

European Patent Office

Office européen des brevets

(19)

(11) Veröffentlichungsnummer: **0 146 863**

**A2**

(12) **EUROPÄISCHE PATENTANMELDUNG**

(21) Anmeldenummer: **84115112.9**

(22) Anmeldetag: **10.12.84**

(51) Int. Cl.⁴: **A 61 K 31/60**
**A 61 K 9/22, A 61 K 47/00**

(30) Priorität: **23.12.83 DE 3346571**

(43) Veröffentlichungstag der Anmeldung:
**03.07.85 Patentblatt 85/27**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Streuff, Bernhard, Dr.**
**Morgengraben 10**
**D-5000 Köln 80(DE)**

(72) Erfinder: **PÜtter, Johann, Prof. Dr.**

**verstorben(DE)**

(54) **Orale retardierte Acetylsalicylsäureformulierungen.**

(57) Die vorliegende Erfindung betrifft feste orale Acetylsalicylsäure-Formulierungen mit verzögerter Freisetzung und lang-andauernder Wirkung sowie deren Herstellung.

EP 0 146 863 A2

Croydon Printing Company Ltd

BAYER AKTIENGESELLSCHAFT          5090 Leverkusen, Bayerwerk

Konzernverwaltung RP
Patentabteilung                   Si/by-c


## Orale retardierte Acetylsalicylsäureformulierungen

Die vorliegende Erfindung betrifft feste orale Acetylsalicylsäure-Formulierungen mit verzögerter Freisetzung des Wirkstoffs und lang-andauernder Wirkung sowie deren Herstellung.

Die für eine lang-andauernde therapeutische Wirkung nötige Höhe und Konstanz der Blutspiegel-Werte eines Arzneistoffes hängen von der physiologischen Halbwertszeit sowie der Auflösung und Resorption aus der Arzneiform ab. Die Auflösegeschwindigkeit läßt sich über die Löseparameter des Wirkstoffes (Teilchengröße, Derivate) oder die Freisetzung aus der Arzneiform in den Verdauungssäften steuern.

Eine für eine Langzeit-Wirkung nötige verzögerte Freisetzung ist meistens nur mit aufwendigen galenischen Maßnahmen wie retardierender Umhüllung von Kristallen, Pellets, Tabletten oder Kapseln, Matrixtabletten, osmotischen Systemen etc. zu erreichen.

Le A 22 020 -Ausland

0146863

Für die Indikation "Behandlung akuter Schmerzzustände" wird in den Arzneibüchern für Acetylsalicylsäure-Präparate ein schneller Zerfall und eine rasche Freisetzung gefordert. Dieses wird zumeist dadurch erreicht, daß der Wirkstoff ohne Bindemittel mit einem oder mehreren Hilfsstoffen, die den Zerfall der Zubereitung fördern (z.B. Maisstärke), zu Tabletten oder Kapseln verarbeitet wird.

Dagegen erfordert die Thromboseprophylaxe mittels der Thrombocytenaggregations-Hemmung der Acetylsalicylsäure eine verzögerte, gleichmäßige Freigabe des Wirkstoffes, die z.B. bei der Spezialität Colfarit [R] Tabl. 0,5 g durch eine Mikroverkapselung der ASS-Kristalle erreicht wird. Dieses Verfahren ist aber sehr aufwendig.

Aufgabe der vorliegenden Erfindung war es, eine Acetylsalicylsäure-Zubereitung mit retardierendem Effekt zu finden, die kostengünstig herzustellen ist und sich zudem durch verbesserte chemische und mechanische Stabilität wie auch durch gute Magenverträglichkeit auszeichnen soll.

Die vorliegende Erfindung betrifft nun ein äußerst einfaches Herstellungsverfahren für retardierte feste Acetylsalicylsäure-Formulierungen, die nur aus Wirk- und einfachen Hilfsstoffen bestehen und sich zeitabhängig vollständig auflösen sowie die Acetylsalicylsäure-Formulierungen als solche und ihre Verwendung als feste Retard-Formulierungen.

Le A 22 020

Gegenstand der vorliegenden Erfindung ist daher eine
Acetylsalicylsäure-Formulierung enthaltend

60-90 Gew.-% Acetylsalicylsäure,
8-40  Gew.-% vorverkleisterte Stärke sowie
ggf. 0-30  Gew.-% Zuschlagstoffe,
vorzugsweise 70-88 Gew.-% Acetylsalicylsäure und
12-30 Gew.-% vorverkleisterte Stärke.

Besonders bevorzugt enthält die ASS-Formulierung 70 -
80 Gew.-% Acetylsalicylsäure.

Ein weiterer Gegenstand der vorliegenden Erfindung ist
ein Verfahren zur Herstellung retardierend wirkender
Formulierungen, die Acetylsalicylsäure, vorverkleisterte Stärke und ggf. Zuschlagstoffe enthalten, bei dem die
einzelnen Bestandteile zu einer Tablettenmischung, insbesondere zu einem Granulat verarbeitet werden, gemischt
und vorzugsweise direkt zu Tabletten verpreßt werden.

Man wendet dabei bevorzugt eine Trockengranulierung an.
Im einzelnen seien Brickettierungen und Walzengranulierungen
genannt. Alternativ kann diese Mischung  auch in Hartgelatine-
kapseln abgefüllt werden. Die Hartgelatine kann bei Bedarf
gefärbte und/oder farbgebende sowie Geschmacksstoffe enthalten.

Ferner gehört zur vorliegenden Erfindung die Verwendung
von Acetylsalicylsäure-Formulierungen, bestehend aus
Acetylsalicylsäure, vorverkleisterter Stärke sowie ggf.
Zuschlagstoffen als retardiert resorbiertes Arzneimittel,
insbesondere als Schmerzmittel, Entzündungshemmer und/
oder bei der Beeinflussung der Blutplättchenaggregation.

Unter vorverkleisterter Stärke im Sinne der vorliegenden
Erfindung wird natürliche Stärke jedweder Herkunft, die

Le A 22 020

durch einen physikalischen oder chemischen Prozeß verkleistert und anschließend getrocknet worden ist, verstanden. Bevorzugt wird unter vorverkleisterter Stärke
eine solche verstanden, welche die Spezifizierung von
NF XV erfüllt. NF XV ist veröffentlicht als "The National
Formulary", fifteenth edition, Official from July 1,
1980, United States Pharmacopical Convention, Inc.

Unter Zuschlagstoffen im Sinne der vorliegenden Erfindung werden verstanden:

Maisstärke (Amylum maylis, Ph. Eur.)
oder andere Stärkearten (Amyla Ph. Eur., Starch
NF XV),
Cellulose Pulver (Cellulosi pulvis, DAB 8 Powdered
Cellulose, NF XV),
Mikrokristalline Cellulose Microcristalline Cellulose, NF XV),
Magnesiumstearat (Magnesii Stearas, Ph. Eur. NF XV),
oder andere pharmazeutische Hilfsstoffe entsprechend
den Spezifikationen der Arzneimittelhandbücher.

Das gefundene Verfahren ermöglicht es, die Freisetzungsgeschwindigkeit von Acetylsalicylsäure aus festen Arzneiformen durch Variation des Anteils an vorverkleisterter
Stärke in sehr breiten Grenzen einzustellen. Dazu wird
die Acetylsalicylsäure mit 2-40 Gew.-%, bevorzugt 5-30
Gew.-%, ganz besonders bevorzugt 10-20 Gew.-% vorverkleisterter Stärke und 0-30 Gew.-% Hilfsstoffen intensiv gemischt und direkt ohne weitere Operationen zu
Tabletten verpreßt oder in Hartgelatine-Kapseln abgefüllt. Es handelt sich somit um ein sehr kostengünstiges
Verfahren zur Herstellung retardierend wirkender fester
oraler Arzneiformen.

Le A 22 020

Die vorverkleisterte Stärke ist in trockenem Zustand ein feines Pulver und läßt sich als solches handhaben. Bei Kontakt mit Wasser oder überwiegend wäßrigen Lösungen quillt sie sehr schnell zu einem thixotropen Gel auf.

Dieser Vorgang läuft so auch in der fertigen Arzneiformulierung ab, wobei die Acetylsalicylsäure-Partikel von dem aufquellenden Gel umhüllt werden. Die Freisetzung der Acetylsalicylsäure erfolgt dann durch Diffusion der gelösten Moleküle durch die Gel-Hülle und ist somit von deren Dicke, d.h. von dem Anteil an vorverkleisterter Stärke in der Formulierung, abhängig. Durch verschiedene Einsatzmengen an vorverkleisterter Stärke lassen sich unterschiedliche Freisetzungsgeschwindigkeiten einstellen, die, wie in vivo-Untersuchungen zeigten, zu analogen Abstufungen der Blutspiegelkurven führen. Die Resorption der Acetylsalicylsäure war bei allen getesteten Formulierungen vollständig, wie Wiederfindungsraten von 94-99 % Salicylat in 0-32 Stunden-Urin der Probanden beweisen (vgl. hierzu auch Beispiel 1).

Nachstehend einige Beispiele, welche die vorliegende Erfindung noch näher erläutern sollen.

Le A 22 020

<u>Beispiel 1</u>

Es werden aus den entsprechenden Trockenmischungen
Tabletten folgender Zusammensetzungen hergestellt:

|                              | A     | B     | C     | D     | E     |
|------------------------------|-------|-------|-------|-------|-------|
| Acetylsalicylsäure           | 500,0 | 500,0 | 500,0 | 500,0 | 500,0 |
| vorverkleisterte Maisstärke  | 100,0 | 90,0  | 80,0  | 70,0  | 60,0  |
| Maisstärke                   | -     | 10,0  | 20,0  | 30,0  | 40,0  |
| Tablettengewicht (mg)        | 600,0 | 600,0 | 600,0 | 600,0 | 600,0 |

Bei der Bestimmung der Freisetzungsgeschwindigkeit nach
der Methode der US-Pharmakopoe (Paddle, 900 ml, 0,1 N HCl)
wurden die folgenden Prozentwerte gemessen:

| Minuten | A | B | C | D | E |
|---------|-------|--------|--------|--------|--------|
| 15 | 1,4 % | 2,9 % | 7,0 % | 13,3 % | 19,7 % |
| 30 | 3,5 % | 5,1 % | 11,1 % | 22,0 % | 28,4 % |
| 60 | 9,9 % | 18,5 % | 25,0 % | 38,0 % | 42,5 % |
| 90 | 24,4 % | 33,9 % | 33,0 % | 49,5 % | 55,5 % |
| 120 | 36,8 % | 47,2 % | 52,7 % | 72,0 % | 77,4 % |

An jeweils zwei nüchternen Probanden wurden nach Einnahme einer Tablette der Formulierungen A, C und E der
zeitliche Verlauf der Plasma-Konzentrationen, bestimmt
als Gesamt-Salicylat, und die Resorptionsquote durch
Messung der innerhalb von 32 Stunden mit dem Urin ausgeschiedenen Salicylsäure-Menge ermittelt:

<u>Le A 22 020</u>

| Minuten | A | C | E | $\underline{/}\overline{m}g/\underline{1}\overline{/}$ |
|---------|------|-------|-------|---|
| 15 | 0,46 | 2,62 | 3,10 | |
| 30 | 1,67 | 5,31 | 11,25 | |
| 60 | 2,88 | 7,10 | 26,71 | |
| 120 | 8,59 | 10,73 | 53,79 | |
| 180 | 11,39 | 14,39 | 49,43 | |
| 0-32˙h | 476,8 mg | 472,1 mg | 494,2 mg | |
| ≙ | 96,7 % | 94,4 % | 98,9 % | |

Die starke Abhängigkeit der Freisetzungsgeschwindigkeit
von dem Anteil an vorverkleisterter Stärke in den Tabletten
und die direkte Korrelierbarkeit mit den resultierenden
Blutspiegeln konnte somit eindeutig belegt werden.

**Beispiel 2**

Aus Tabletten der Zusammensetzung

| | F | G | H |
|---|------|------|------|
| Acetylsalicylsäure | 500,0 | 500,0 | 500,0 |
| vorverkleisterte Maisstärke | 100,0 | 80,0 | 75,0 |
| Cellulose Pulver | | 10,0 | 15,0 |
| Maisstärke | | 10,0 | 10,0 |
| Tablettengewicht $\underline{/}\overline{m}\underline{g}\overline{/}$ | 600,0 | 600,0 | 600,0 |

wurden folgende Freisetzungsraten bestimmt:

| Minuten | F | G | H |
| --- | --- | --- | --- |
| 15 | 5,1 % | 14,8 % | 28,1 % |
| 30 | 12,1 % | 20,1 % | 36,8 % |
| 60 | 24,1 % | 35,3 % | 62,7 % |
| 90 | 39,7 % | 53,3 % | 82,5 % |
| 120 | 51,2 % | 70,9 % | 89,2 % |

Eine pharmakokinetische Studie an sechs männlichen, nüchternen Probanden ergab nach Einnahme jeweils einer Tablette der Formulierungen G und H folgende mittlere Blutspiegel an Acetylsalicylsäure und Salicylsäure:

| Zeit /Std7 | Formul. G | | Formul. H | |
| --- | --- | --- | --- | --- |
| | ASS /mg/l7 | SAS /mg/l7 | ASS /mg/l7 | SAS /mg/l7 |
| 0.25 | 0,548 | 1,530 | 2,522 | 2,962 |
| 0.50 | 1,135 | 5,362 | 4,000 | 15,845 |
| 0.75 | 1,043 | 8,205 | 2,483 | 22,260 |
| 1.00 | 1,002 | 9,618 | 1,958 | 25,428 |
| 1.33 | 1,225 | 12,337 | 1,693 | 28,028 |
| 1.67 | 1,320 | 14,220 | 2,880 | 38,710 |
| 2.00 | 1,612 | 17,802 | 3,636 | 47,688 |
| 3.00 | 1,443 | 27,782 | 1,868 | 57,767 |
| 4.00 | 0,988 | 32,997 | 1,392 | 54,385 |
| 6.00 | 0,095 | 30,137 | 0,157 | 39,462 |
| 8.00 | – | 19,043 | – | 21,810 |
| 24.00 | – | 0,332 | – | 0,383 |

Le A 22 020

Die Resorption (Wiederfindungsrate von Salicylat im
0-32 Stunden-Urin) war bei beiden Formulierungen und
allen Probanden mit 95-97 % vollständig.

Beispiel 3

Eine Mischung bestehend aus 50 Teilen Acetylsalicylsäure,
7 Teilen vorverkleisterter  Maisstärke, 2 Teilen Cellulose-
Pulver und 1 Teil Maisstärke wird auf einer Tablettenpresse
brikettiert. Die Briketts werden wieder zerkleinert, gemischt
und zu den endgültigen Tabletten verpresst.

Beispiel 4

Eine Mischung bestehend aus 50 Teilen Acetylsalicylsäure,
8 Teilen vorverkleisterter Maisstärke, 1 Teil Cellulose-
Pulver und 1 Teil Maisstärke wird auf einer Walzenpresse
bei einem Walzendruck zwischen 20 und 50 bar trockengranuliert. Das Granulat wird zerkleinert, gemischt und zu
Tabletten verpresst.

Le A 22 020

0146863

Patentansprüche

1.  Acetylsalicylsäure-Formulierung mit Retardwirkung,
    enthaltend
    60-90 Gew.-% Acetylsalicylsäure,
     8-40 Gew.-% vorverkleisterte Stärke sowie ggf.
    0-30 Gew.-% Zuschlagstoffe.

2.  Acetylsalicylsäure-Formulierung nach Anspruch 1,
    enthaltend
    70-88 Gew.-% Acetylsalicylsäure und
    12-30 Gew.-% vorverkleisterte Stärke.

3.  Acetylsalicylsäure-Formulierung nach Anspruch 1
    oder 2 zur Verwendung bei der Bekämpfung von Krankheiten.

4.  Acetylsalicylsäure-Formulierung nach Anspruch 1
    oder 2 zur Verwendung bei der Bekämpfung von
    Schmerzen, Entzündungen oder Blutplättchenaggregation.

5.  Verfahren zur Herstellung von retardiert wirkender
    Formulierungen der Acetylsalicylsäure,
    dadurch gekennzeichnet, daß man
    60 - 90 Gew.-% Acetylsalicylsäure zusammen mit
     8 - 40 Gew.-% vorverkleisterter Stärke und gegebenenfalls
     0 - 30 Gew.-% Zuschlagstoffen
    zu einer Tablettenmischung; insbesondere zu einem Granulat
    verarbeitet.

6.  Verfahren nach Anspruch 5, dadurch gekennzeichnet,
    daß man eine Trockengranulierung durchführt.

Le A 22 020

7. Verfahren nach Anspruch 6, dadurch gekennzeichnet, daß man eine Brickettierung durchführt.

8. Verfahren nach Anspruch 6, dadurch gekennzeichnet, daß man eine Walzengranulierung durchführt.

9. Verwendung von Acetylsalicylsäure-Formulierungen nach Anspruch 1 oder 2 bei der Bekämpfung von Krankheiten.

10. Verwendung von Acetylsalicylsäure-Formulierungen nach Anspruch 1 oder 2 als Schmerzmittel, Entzündungshemmer oder bei der Beeinflussung der Blutplättchenaggregation.

Le A 22 020